# EUROPEAN PATENT SPECIFICATION

(11) **EP 2 350 641 B1**
(45) Date of publication and mention of the grant of the patent: **24.09.2014**
(21) Application number: 09814951.1
(22) Date of filing: 05.08.2009
(51) Int. Cl.: A61K 38/17, A61K 31/52, A61K 39/395, A61P 35/00, A61K 45/06

(54) **A COMBINATION OF A TNF ALPHA INHIBITOR, AN IAP INHIBITOR AND A TRAIL RECEPTOR AGONIST FOR USE IN TREATING CANCER**
EINE KOMBINATION AUS EINEM TNF-ALPHA-HEMMER, EINEM IAP-BLOCKER UND EINEM AGONISTEN DES TRAIL-REZEPTORS ZUR VERWENDUNG IN DER BEHANDLUNG VON KREBS
UN COMBINASION D'UN INHIBITEUR DE TNF ALPHA, UN INHIBITEUR DE PAI ET UN AGONISTE DU RÉCEPTEUR TRAIL POUR UTILISATION DANS LE TRAITEMENT DU CANCER

(30) Priority: 22.09.2008 US 99088 P
(43) Date of publication of application: 03.08.2011
(73) Proprietor: Amgen Inc., Thousand Oaks, CA 91320-1799 (US); Tetralogic Pharmaceuticals Corporation, Malvern, PA 19355 (US)
(72) Inventor: HOLLAND, Pamela, M., Thousand Oaks, CA 91320-1799 (US); PIASECKI, Julia, C., Thousand Oaks, CA 91320-1799 (US); BENETATOS, Christopher, A., Malvern, PA 19355 (US); CHUNDURU, Srinivas, K., Malvern, PA 91355 (US); MCKINLAY, Mark, A., Malvern, PA 19355 (US)
(74) Representative: Wilson, Justin Scott
(86) International application number: PCT/US2009/052789
(87) International publication number: WO 2010/033315

(56) References cited:
- WO-A1-2007/101347
- WO-A2-2005/123772
- US-A1- 2005 079 172
- US-A1- 2006 194 741
- US-A1- 2007 243 188
- US-A1- 2008 089 896
- US-A1- 2008 132 485

## Description

### FIELD OF THE INVENTION

This invention is in the field of cancer treatment.

### BACKGROUND OF THE INVENTION

The Inhibitor of Apoptosis Proteins (IAPs) are a family of proteins that suppress apoptosis via unique mechanisms. For example, X-linked inhibitor of apoptosis protein (XIAP) prevents apoptosis by sequestering and blocking the activity of both the initiator and effector caspases. Caspases are cysteine-dependent aspartyl-specific proteases that function to cleave cellular substrates during the apoptosis process. In contrast, other IAP family members such as cellular inhibitor of apoptosis protein 1 (cIAP1) and cellular inhibitor of apoptosis protein 2 (cIAP2) prevent apoptosis that is induced through tumor necrosis factor α (TNFα) and activation of the TNFα receptor 1 (TNFR1) signaling pathway. Despite unique mechanisms for blocking apoptosis, all IAP members contain one or more zinc-binding baculoviral repeat (BIR) domains, which mediate protein-protein interactions and anti-apoptotic function.

The mitochondrial protein Smac (second mitochondrial activator of caspases) is an endogenous regulator of IAP proteins and is released into the cytosol during apoptosis. Smac can bind with high affinity to the BIR3 domain of cIAP1, cIAP2, and XIAP. The pro-apoptotic activity of Smac depends on a four amino acid IAP-binding motif located at the N terminus of the mature protein. This motif, Ala-Val-Pro-Ile, is sufficient for binding the BIR3 domain of cIAP1, cIAP2, and XIAP and competes for caspase 9 binding at this site. Smac-IAP interactions thereby prevent IAPs from inhibiting caspase 9.

There is a continuing need in the art for cancer treatments involving IAP inhibitors.

### BRIEF DESCRIPTION OF THE FIGURES

**FIG. 1****.** Graph demonstrating that compound X-induced serum caspase 3/7 activity in nude, non-tumor bearing mice is reduced by inhibition of TNFα. On the X-axis, the "-" and "+" below each bar indicates whether or not compound X was added at a dosage of 15 mg/kg; the numbers below each bar indicate the mouse group; and the lines under the numbers group the mouse groups according to their pretreatment (abbreviations are explained in Example 1). The Y axis is caspase 3/7 activity expressed as relative light units (RLU).
**FIG. 2****.** Graph demonstrating bodyweight changes in wild-type and TNF receptor double knock-out mice following treatment with compound X. Abbreviations on the X-axis are explained in Example 2.
**FIG. 3****.** Graph demonstrating that compound X-induced serum caspase-3 levels are reduced in TNF receptor double knock-out mice. Abbreviations on the X-axis are explained in Example 2. The Y-axis indicates serum caspase 3/7 levels expressed as RLU per second.
**FIG. 4****.** Graph demonstrating that decreases in platelet counts induced by compound X are greater in wild-type than in TNF receptor double knock-out mice. Abbreviations on the X-axis are explained in Example 2. The numbers on the Y-axis are the numbers (x 10³) of platelets per µl.
**FIG. 5****.** Graph demonstrating that decreases in reticulocyte counts induced by compound X are greater in wild-type than in TNF receptor double knock-out mice. Abbreviations on the X-axis are explained in Example 2. The numbers on the Y-axis are the numbers (x 10⁹) of reticulocytes per µl.
**FIG. 6****.** Graph demonstrating that compound X-induced small intestine crypt cell apoptosis is greater in wild-type than in TNF receptor double knock-out mice. Abbreviations on the X-axis are explained in Example 2. The Y-axis indicates the apoptosis grade (0-5) as explained in Example 2.
**FIG. 7****.** Hematoxylin- and eosin-stained histological sections demonstrating that compound X-induced small intestine crypt cell apoptosis is greater in wild-type than in TNF receptor double knock-out mice. **FIG. 7A****,** section of a small intestine of a wild-type mouse on day 5 after treatment with compound X (see Example 2). **FIG. 7B****,** section of a small intestine of a TNF receptor double knock-out mouse on day 5 after treatment with compound X (see Example 2).
**FIG. 8****.** Graph demonstrating that compound X-induced epidermal basal cell apoptosis is greater in wild-type than in TNF receptor double knock-out mice. Abbreviations on the X-axis are explained in Example 2. The Y-axis indicates the apoptosis grade (0-5) as explained in Example 2.
**FIG. 9****.** Hematoxylin- and eosin-stained histological sections demonstrating that compound X-induced epidermal basal cell apoptosis is greater in wild-type than in TNF receptor double knock-out mice. **FIG. 9A****,** section of basal epithelial cells of the skin of a wild-type mouse on day 5 after treatment with compound X (see Example 2), showing mild apoptosis. **FIG. 9B****,** section of basal epithelial cells of the skin of a TNF receptor double knock-out mouse on day 5 after treatment with compound X (see Example 2), showing that no apoptosis is present.
**FIG. 10****.** Graph demonstrating that compound X-induced thymic cortical atrophy is greater in wild-type than in TNF receptor double knock-out mice. Abbreviations on the X-axis are explained in Example 2. The Y-axis indicates the apoptosis grade (0-5) as explained in Example 2.
**FIG. 11****.** Hematoxylin- and eosin-stained histological sections demonstrating that compound X-induced thymic cortical atrophy is greater in wild-type than in TNF receptor double knock-out mice. **FIG. 11A**, thymus of a wild-type mouse on day 5 after treatment with compound X (see Example 2), showing thymic cortical atrophy. **FIG. 11B**, thymus of a TNF receptor double knock-out mouse on day 5 after treatment with compound X (see Example 2), showing that the thymic cortex is not atrophied and is normal in appearance.
**FIG. 12A****.** Graph demonstrating body weight changes in wild-type and TNF receptor double knock-out mice following treatment with compound X. Abbreviations on the X-axis are explained in Example 3.

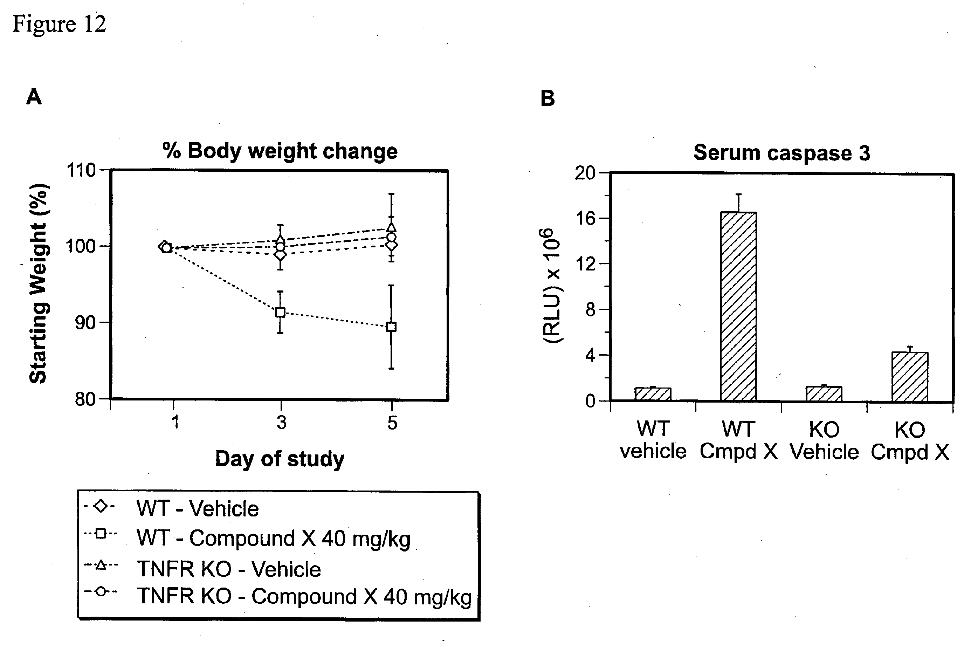

**FIG. 12****B.** Graph demonstrating that compound X-induced serum caspase-3 levels are reduced in TNF receptor double knock-out mice. Abbreviations on the X-axis are explained in Example 4. The Y-axis indicates serum caspase 3/7 levels expressed as relative light units (RLU).

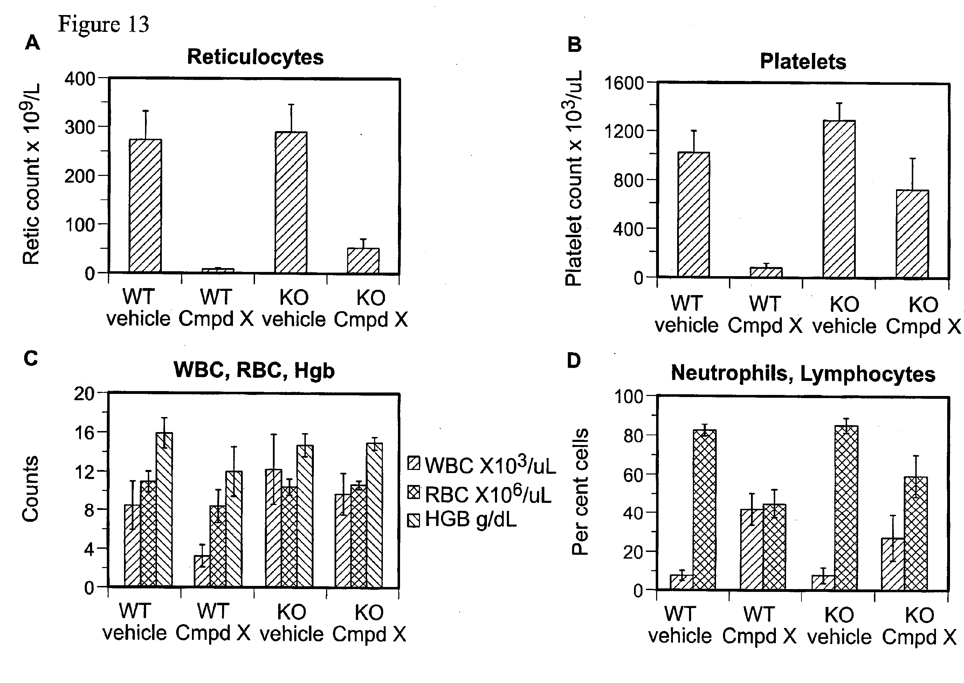

**FIG. 13A****.** Graph demonstrating that decreases in reticulocyte counts induced by compound X are greater in wild-type than in TNF receptor double knock-out mice. Abbreviations on the X-axis are explained in Example 4. The numbers on the Y-axis are the numbers (x 10⁹) of reticulocytes per µl.
**FIG. 13****B.** Graph demonstrating that decreases in platelet counts induced by compound X are greater in wild-type than in TNF receptor double knock-out mice. Abbreviations on the X-axis are explained in Example 4. The numbers on the Y-axis are the numbers (x 10³) of platelets per µl.
**FIG. 13C****.** Graph demonstrating that decreases in white blood cell counts induced by compound X are greater in wild-type than in TNF receptor double knock-out mice. Red blood cell counts and hemoglobin levels are relatively unchanged in wild-type vs TNF receptor double knock-out mice. Abbreviations on the X-axis are explained in Example 4. The numbers on the Y-axis are the relative counts of each marker measured as outlined on the graph legend.
**FIG. 13D****.** Graph demonstrating that increases in neutrophil counts and decreases in lymphocyte counts induced by compound X are relatively equivalent in both wild-type and TNF receptor double knock-out mice. Abbreviations on the X-axis are explained in Example 4. The numbers on the Y-axis are the percentage (%) of each cell type present.

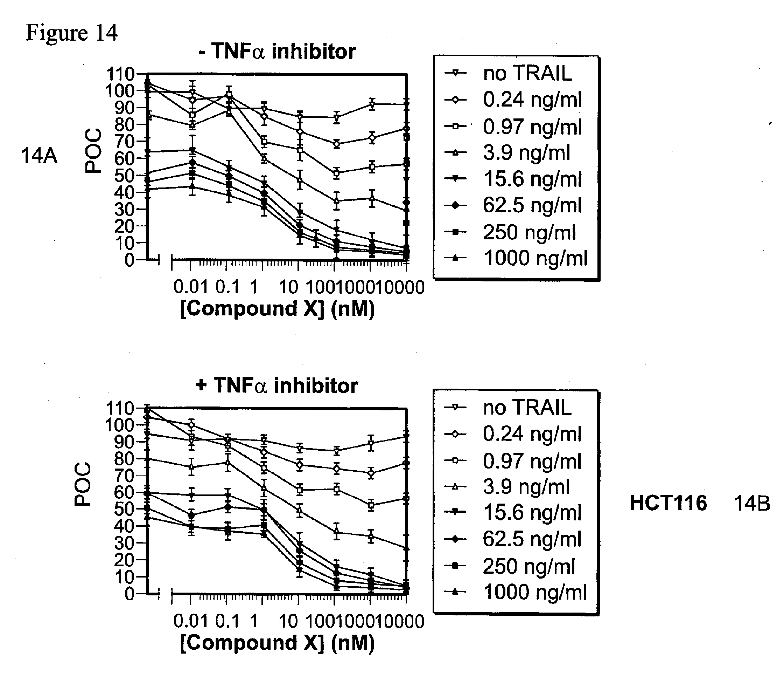

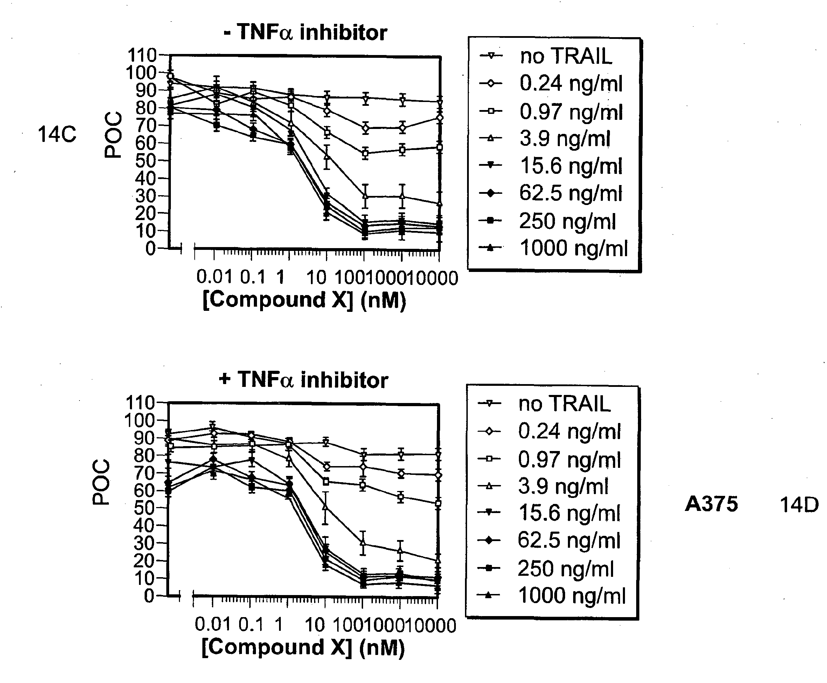

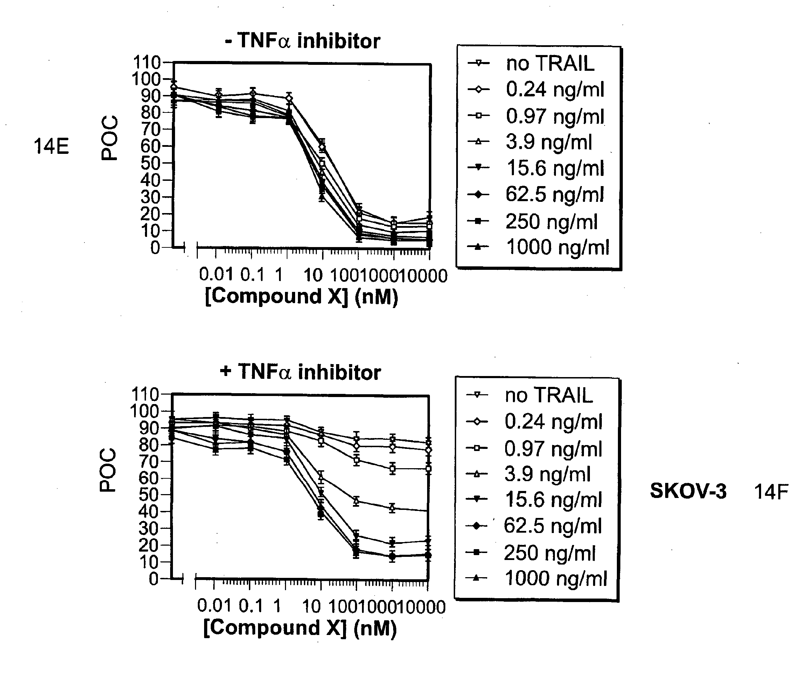

**FIG. 14****.** Graphs demonstrating viability of HCT116 colon cancer cells **(****FIGS. 14A, B**), A375 melanoma cells **(****FIGS. 14C, D**), and SKOV-3 ovarian cancer cells **(****FIGS. 14E, F**) after treatment with an IAP inhibitor ("compound X", shown in Example 1) and the effects of TNFα inhibition on the synergy of TNF-related apoptosis-inducing ligand (TRAIL) and compound X *in vitro.* The X-axis of each graph is the concentration of compound X in nM. The Y-axis is the percent of surviving cells compared with control (untreated) cells ("POC," percent of control). Concentrations of TRAIL are indicated at the right of each graph.

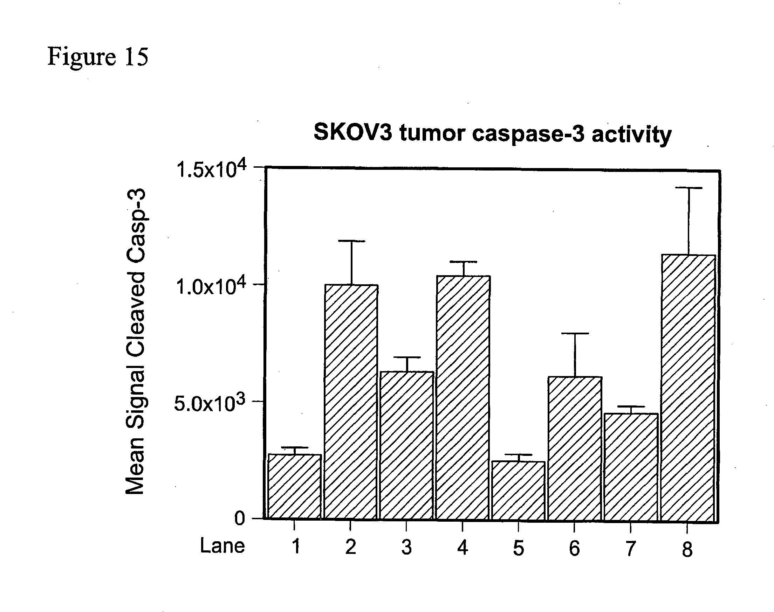

**FIG. 15****.** Graph showing the effects on tumor caspase-3 activity after various treatments of tumor-bearing mice. See Example 5.

### SUMMARY OF THE INVENTION

The invention is defined by the claims.

The invention provides a combination of (a) a TNFα inhibitor; (b) an IAP inhibitor; and (c) a TRAIL receptor agonist. For use in treating cancer, wherein the TNFα inhibitor can be administered for a sufficient amount of time to achieve inhibition of TNFα prior to administration of the IAP inhibitor and the TRAIL receptor agonist. Each of the IAP inhibitor, the TNFα inhibitor, and the TRAIL receptor agonist can, but need not, be a small molecule. "Small molecule" as used herein is a synthetic or semi-synthetic organic chemical compound having a molecular weight of less than about 2000 Daltons.

The IAP inhibitor and the TRAIL receptor agonist can be administered simultaneously or sequentially. In some embodiments the cancer is ovarian cancer, breast cancer, or colon cancer.

In some embodiments the IAP inhibitor is a Smac mimetic. The TNFα inhibitor can be, for example, an antibody, such as Infliximab (*e.g.*, REMICADE^{®} infliximab) and Adalimumab (*e.g*., HUMIRA^{®} adalimumab), or can be a receptor-construct fusion protein, such as Etanercept (*e.g*., ENBREL^{®} etanercept).

In some embodiments the TRAIL receptor agonist is an antibody, such as HGS-ETR1 or "mapatumumab" and HGS-ETR2 or "lexatumumab" (Human Genome Sciences; Georgakis et al., Br J Haematol. 2005 Aug;130(4):501-10), HGS-TR2J (Human Genome Sciences), Apomab (Genentech; Adams et al., Cell Death Differ. 2008 Apr;15(4):751-61), CS-1008 (Daiichi Sankyo; see Yada et al., Ann Oncol. 2008 Jun; 19(6):1060-7), LBY135 (Novartis; Natoni et al., Br J Haematol. 2007 Nov;139(4):568-77), and TRA-8 (University of Alabama-Birmingham; Ichikawa et al., Nat Med. 2001 Aug;7(8):954-60). The antibody can be a DR4 antibody, a DR5 antibody, or an antibody which binds to both DR4 and DR5. In other embodiments the TRAIL receptor agonist is recombinant human TRAIL. In other embodiments the TRAIL receptor agonist is a peptibody (a molecule comprising an antibody Fc domain attached to at least one peptide). The production of peptibodies is generally described in PCT publication WO 00/24782, published May 4, 2000. See US 2008/0213277. In other embodiments the TRAIL receptor agonist is an avimer. See US 2006/0286603 and 2006/0223114.

### DETAILED DESCRIPTION OF THE INVENTION

Applicants have discovered that treatment with a TNFα inhibitor in conjunction with the combination therapy of an IAP inhibitor and a TRAIL receptor agonist effectively induces apoptosis while curtailing or attenuating the risk of unwanted effects. Methods of the invention can result in a broader range of anti-tumor activity and an improved therapeutic index, *e.g.,* for IAP inhibitors.

### IAP inhibitors

An "IAP inhibitor" according to the invention is an agent that binds to an IAP BIR domain. Binding to an IAP BIR domain is determined substantially as described by Nikolovska-Coleska et al., Analytical Biochemistry 332, 261-73, 2004, using as the fluorogenic substrate the fluorescently labeled peptide AbuRPF-K(5-Fam)-NH₂. The binding affinities of the compounds are reported as a K_{D} value (µm). Briefly, an agent is mixed with 5 nM of the fluorescently labeled peptide (i.e., a mutated N-terminal Smac peptide - AbuRPF-K(5-Fam)-NH₂) and 40 nM of the respective BIR domain for 15 minutes at room temperature in 100 mL of 0.1M potassium phosphate buffer, pH 7.5 containing 100 mg/ml bovine γ-globulin. Following incubation, the polarization values (mP) are measured using a 485 nm excitation filter and a 520 nm emission filter. An agent which binds to an IAP BIR domain with a K_{d} of 10 µM or less, preferably 1 mM of less is an IAP inhibitor according to the invention.

IAP inhibitors can be small molecules or can be peptides or protein-based molecules. In some embodiments an IAP inhibitor is a Smac mimetic. Examples of IAP inhibitors useful in the invention include, but are not limited to, those disclosed in US 2006/0128632; US 2006/0052311; US 2005/0234042; WO 2006/133147; WO 2008/045905; WO 2008/016893; US 2005/0261203; US 2006/0014700; US 2006/0167066; WO 2006/014361; WO 2005/094818; WO 2007/106192; US 2008/0146808; US 2007/0299052; 7,345,081; 7,244,851; US 2005/0261203; US 2006/0167066; US 2006/0014700; US 2006/0025347; US 2006/0194741; US 2007/0042428; WO 2006/091972; WO 2007/021825; US 2008/0021066; US 2006/0194741; US 2008/0020986; WO 2005/069888; WO 2005/069894; WO 2006/010118; WO 2007/130626; WO 2006/017295; WO 2006/128455; WO 2006/020060; WO 2007/136921; US 2008/0171693; 7,342,093; US 2007/0032437; US 2005/0227302; US 2004/0171105; US 2005/0233411; US 2002/0132786, US 2003/0073629; and US 2005/0176649.

### TRAIL preceptor agonists

A TRAIL receptor agonist is an agent that binds to a TRAIL receptor, such as TRAIL receptor 1 (TRAIL R1, also known as "death receptor 4" or DR4), TRAIL receptor 2 (TRAIL R2, also known as "death receptor 5" or DR5), or both DR4 and DR5, and leads to apoptosis in at least one one mammalian (e.g., human) cell type (such as a TRAIL-sensitive tumor cell line) when used in an amount effective to induce apoptosis under physiological conditions. TRAIL receptor agonists according to the invention do not bind to TRAIL decoy receptors. TRAIL receptor agonists according to the invention encompass antibodies which require cross-linking for *in vitro* apoptotic activity.

Binding to a TRAIL receptor can be assessed by flow cytometry. Functional activity of a TRAIL receptor agonist can be monitored by measurement of apoptosis induction in Colo205 cells using standard cell viability and caspase activation assays. In some embodiments, TRAIL receptor agonists bind to DR4 and/or DR5 as assessed by flow cytometry and result in a 60-100% decrease in cell viability and a 5-12 fold increase in caspase 3 activity. In some embodiments the TRAIL receptor agonist is a soluble TRAIL polypeptide.

### Flow cytometry

Binding of an agent to a TRAIL receptor is detected by flow cytometry. In one embodiment, Colo205 cells are incubated in blocking buffer (10% FBS/5% goat serum/FACS buffer), washed in FACS buffer (1%FBS/PBS), and resuspended in 10 µg/ml primary antibody (DR4 orTRAIL-R1 and/or DR5 or TRAIL-R2 antibodies, which are commercially available, *e.g*. from eBiosciences, San Diego, CA) or isotype control for 45 min at 4°C. After washing, cells are resuspended in 10 µg/ml biotinylated secondary antibody for 45 min at 4°C, washed again and incubated with 10 µg/ml streptavidin-phycoerythrin (BD Pharmingen, San Diego, CA) for 45 min at 4°C. Cells are then analyzed on a flow cytometer. TRAIL receptor agonist binding may also be shown by measuring binding affinity of an agonist to the extracellular domain of DR4 (TRAIL-R1) and/or DR5 (TRAIL-R2) protein immobilized on a 96-well plate using an ELISA based format, as described in (Pukac et al., Br. J. Cancer 92, 1430-41, 2005).

### Measurement of apoptosis induction

To monitor cell viability, Colo205 cells are plated at 2 x 10⁴ cells/well and treated with a TRAIL receptor agonist at a range of concentrations relevant for the modality of the agonist (antibody, recombinant protein) as described in Gliniak, Cancer Res. 59, 6153-58, 1999; Motoki et al., Clin. Cancer Res. 11, 3126-35, 2005; or *Pukac et al.,* 2005. After 24-48 hours, viability is assessed by detecting the percentage of dead cells in a well.

### Caspase 3 activity assay

For a caspase 3 activity assay, Colo205 cells are plated at 1.8 x 10⁴ cells/well and treated with a TRAIL receptor agonist at a range of concentrations relevant for the modality of the agonist (antibody, recombinant protein) as described in Gliniak, Cancer Res. 59, 6153-58, 1999; Motoki et al., Clin. Cancer Res. 11, 3126-35, 2005; or Pukac *et al.,* 2005. After 5-18 hr, caspase 3 activity is measured (for example, using the Caspase Glo 3/7 assay (Promega, Madison, WI) according to manufacturer's protocol).

TRAIL receptor agonists include TRAIL itself (including recombinant TRAIL), as well as antibodies (particularly agonist monoclonal antibodies), peptibodies, avimers, peptide-mimetic compounds, small molecules, and proteins. The monoclonal antibodies HGS-ETR1 ("mapatumumab," DR4 antibody), HGS-ETR2 ("lexatumumab," DR5 antibody), HGS-TR2J (DR5 antibody), Apomab (human DR5 antibody), recombinant human Apo2L/TRAIL (which activates both DR4 and DR5), CS-1008 (humanized DR5 antibody), AMG 655 (DR5 antibody), LBY135 (DR5 antibody), and TR8 (DR5 antibody) are examples of TRAIL receptor agonists. See also U.S. Patent 7,115,717; U.S. Patent 7,361,341; US 2007/0292411; US 2005/0249729; and US 2002/0155109.

### TNFα inhibitors

A TNFα inhibitor according to the invention is an agent which decreases the ability of TNFα to kill cells by 60-100%, which can be assayed as described in Mohler et al., J Immunol. 1993 Aug 1; 151(3): 1548-61. TNFα inhibitors typically inhibit or block at least one biological activity of TNFα, such as preventing TNF from binding to a TNF receptor, blocking production of TNFα by intracellular processing (*e.g*., by blocking transcription, translation, or post-translational modification, expression on the cell surface, secretion, or assembly of the bioactive trimer of TNFα). In some embodiments TNFα inhibitors act by inducing regulation of metabolic pathways such as those involving the up or down regulation of TNFα production. In other embodiments TNFα inhibitors modulate cellular sensitivity to TNFα by decreasing such sensitivity.

TNFα inhibitors can be antibodies, peptibodies, avimers, peptide-mimetic compounds, small molecules, or proteins. Screening methods which can be used to determine the inhibitory effect of a TNFα inhibitor include *in vitro* and *in vivo* assays. *In vitro* assays include a TNFα cytotoxicity assay, such as a radioimmunoassay, which determines a decrease in cell death by contact with TNFα; and assays for TNFα induction of cytokines. Cell death can be determined using ID₅₀ values which represent the concentration of a TNFα inhibitor which decreases the cell death rate by 50%.

TNFα inhibitors include, but are not limited to, broad spectrum immunosuppressants (*e.g*., steroids, including synthetic glucocorticoids such as dexamethasone), curcumin, antibodies, and fusion constructs. Antibodies such as Infliximab (REMICADE^{®} infliximab), Adalimumab (HUMIRA^{®} adalimumab), and receptor-construct fusion proteins such as Etanercept (ENBREL^{®} etanercept) are examples of TNFα inhibitors. See also TNFα inhibitors disclosed in US 2008/0033149; US 2008/0167223; and US 2007/0141057. TNFα inhibitors also include antibodies and other agents which bind to the TNF receptor, thereby inhibiting biological effects of TNFα.

### IAP inhibitors, TNFα inhibitors, and/or TRAIL receptor agonists which are small molecules

IAP inhibitors, TNFα inhibitors, and TRAIL receptor agonists which are small molecules can be administered as pharmaceutically acceptable salts, esters, amides or prodrugs.

The term "salts" refers to inorganic and organic salts of compounds of the present invention. The salts can be prepared in situ during the final isolation and purification of a compound, or by separately reacting a purified compound in its free base or acid form with a suitable organic or inorganic base or acid and isolating the salt thus formed. Representative salts include the hydrobromide

hydrochloride, sulfate, bisulfate, nitrate, acetate, oxalate, palmitiate, stearate, laurate, borate, benzoate, lactate, phosphate, tosylate, citrate, maleate, fumarate, succinate, tartrate, naphthylate, mesylate, glucoheptonate, lactobionate, and laurylsulphonate salts, and the like. The salts may include cations based on the alkali and alkaline earth metals, such as sodium, lithium, potassium, calcium, magnesium, and the like, as well as non-toxic ammonium, quaternary ammonium, and amine cations including, but not limited to, ammonium, tetramethylammonium, tetraethylammonium, methylamine, dimethylamine, trimethylamine, triethylamine, ethylamine, and the like. See, for example, S. M. Berge, et al., "Pharmaceutical Salts," J Pharm Sci, 66: 1-19 (1977).

Examples of pharmaceutically acceptable esters include C₁ -C₈ alkyl esters. Acceptable esters also include C₅ -C₇ cycloalkyl esters, as well as arylalkyl esters such as benzyl. C₁ - C₄ alkyl esters are commonly used. Esters of compounds may be prepared according to methods that are well known in the art.

Examples of pharmaceutically acceptable amides include amides derived from ammonia, primary C₁ -C₈ alkyl amines, and secondary C₁ -C₈ dialkyl amines. In the case of secondary amines, the amine may also be in the form of a 5 or 6 membered heterocycloalkyl group containing at least one nitrogen atom. Amides derived from ammonia, C₁ -C₃ primary alkyl amines and C₁ -C₂ dialkyl secondary amines are commonly used. Amides of the compounds may be prepared according to methods well known to those skilled in the art.

The term "prodrug" means a compound that is transformed *in vivo* to yield an IAP inhibitor, a TNFα inhibitor, or a TRAIL receptor agonist. The transformation may occur by various mechanisms, such as through hydrolysis in blood. A discussion of the use of prodrugs is provided by T. Higuchi and W. Stella, "Pro-drugs as Novel Delivery Systems," Vol. 14 of the A.C.S. Symposium Series, and in Bioreversible Carriers in Drug Design, Edward B. Roche, ed., American Pharmaceutical Association and Pergamon Press, 1987.

To illustrate, if the compound contains a carboxylic add functional group, a prodrug can comprise an ester formed by the replacement of the hydrogen atom of the acid group with a group such as (C₁-C₈) alkyl, (C₂-Cl₂)alkanoyloxymethyl, 1-(alkanoyloxy)ethyl having from 4 to 9 carbon atoms, 1-methyl-1-(alkanoyloxy)ethyl having from 5 to 10 carbon atoms, alkoxycarbonyloxymethyl having from 3 to 6 carbon atoms, 1-(alkoxycarbonyloxy)ethyl having from 4 to 7 carbon atoms, 1-methyl-1-(alkoxycarbonyloxy)ethyl having from 5 to 8 carbon atoms, N-(alkoxycarbonyl)aminomethyl having from 3 to 9 carbon atoms, 1-(N-(alkoxycarbonyl)aminomethyl having from 4 to 10 carbon atoms, 3-phthalidyl, 4-crotonolactonyl, gamma-butyrolacton-4-yl, di-N,N-(C₁-C₂)alkylamino(C₂-C₃)alkyl (such as β-dimethylaminoethyl), carbamoyl-(C₁-C₂)alkyl, N,N-di(C₁-C₂)alkylearbamoyl-(C₁-C₂)alkyl and piperidino-, pyrrolidino- or morpholino(C₂C₃)alkyl.

Similarly, if a compound comprises an alcohol functional group, a prodrug can be formed by the replacement of the hydrogen atom of the alcohol group with a group such as (C₁ C₆)alkanoyloxymethyl, 1-((C₁-C₆)alkanoyloxy)ethyl, 1-methyl-1-((C₁ C₆)alkanoyloxy)ethyl, (C₁-C₆)alkoxycarbonyloxymethyl, N-(C₁-C₆)alkoxycarbonylaminomethyl, succinoyl, (C₁-C₆)alkanoyl, α-amino(C₁-C₄)alkanoyl, arylacyl and α-aminoacyl, or α-aminoacyl-α-aminoacyl, where each α-aminoacyl group is independently selected from the naturally occurring L-amino acids, -P(O)(OH)₂,-P(O)(O(C₁-C₆)alkyl)₂ or glycosyl (the radical resulting from the removal of a hydroxyl group of the hemiacetal form of a carbohydrate).

An IAP inhibitor, TRAIL receptor agonist, or TNFα inhibitor may contain asymmetric or chiral centers, and therefore, exist in different stereoisomeric forms. It is contemplated that all stereoisomeric forms of such compounds as well as mixtures thereof, including racemic mixtures and non-racemic mixtures, can be used in the present invention. In addition, the present invention contemplates all geometric and positional isomers. For example, if the compound contains a double bond, both the cis and trans forms (designated as S and E, respectively), as well as mixtures, are contemplated.

Mixture of stereoisomers, such as diastereomeric mixtures, can be separated into their individual stereochemical components on the basis of their physical chemical differences by known methods such as chromatography and/or fractional crystallization. Enantiomers can also be separated by converting the enantiomeric mixture into a diasteromeric mixture by reaction with an appropriate optically active compound (*e.g*., an alcohol), separating the diastereomers and converting (*e.g*., hydrolyzing) the individual diastereomers to the corresponding pure enantiomers. Also, some compounds may be atropisomers (*e.g*., substituted biaryls).

IAP inhibitors, TNFα inhibitors, and TRAIL receptor agonists may exist in unsolvated as well as solvated forms with pharmaceutically acceptable solvents such as water, ethanol, and the like. The present invention contemplates and encompasses both the solvated and unsolvated forms.

It is also possible that IAP inhibitors, TNFα inhibitors, and TRAIL receptor agonists may exist in different tautomeric forms. All tautomers of such compounds are contemplated, such as all of the tautomeric forms of an imidazole moiety and all keto-enol or imine-enamine forms of such compounds.

The present invention also contemplates use of isotopically-labeled IAP inhibitors, TNFα inhibitors, and TRAIL receptor agonists in which one or more atoms are replaced by an atom having an atomic mass or mass number different from the atomic mass or mass number usually found in nature. Examples of isotopes that can be incorporated into such compounds include isotopes of hydrogen, carbon, nitrogen, oxygen, phosphorous, fluorine and chlorine, such as ²H, ³H, ¹³C, ¹⁴C, ¹⁵N, ¹⁶O, ¹⁷O, ³¹P, ³²P, ³⁵S, ¹⁸F, and ³⁶Cl.

### Anti-tumor treatments

In one instance other anti-tumor agents or treatments may be used in place of an IAP inhibitor. In one instance the combination of a TNFα inhibitor, a TRAIL receptor agonist, and an IAP inhibitor may be used in conjunction with other anti-tumor agents and treatments. Such treatments include radiotherapy, chemotherapy, hormone therapy, surgery, immunotherapy, therapy with biological anti-tumor agents, and the like.

Anti-tumor agents can be selected from antineoplastic agents, anti-angiogenic agents, chemotherapeutic agents and peptide cancer therapy agents. In yet another instance, the antineoplastic agents are selected from antibiotic-type agents, alkylating agents, antimetabolite agents, hormonal agents, immunological agents, interferon-type agents, kinase inhibitors, miscellaneous agents and combinations thereof. Such pharmaceutically active compounds/agents can be a traditional small organic chemical molecules or can be macromolecules such as a proteins, antibodies, peptibodies, DNA, RNA or fragments of such macromolecules.

Examples of specific pharmaceutically active agents that can be used in the treatment of cancers and that can be used in combination with methods of the present invention include: methotrexate; tamoxifen; fluorouracil; 5-fluorouracil; hydroxyurea; mercaptopurine; cisplatin; carboplatin; daunorubicin; doxorubicin; etoposide; vinblastine; vincristine; pacitaxel; thioguanine; idarubicin; dactinomycin; imatinib; gemcitabine; altretamine; asparaginase; bleomycin; capecitabine; carmustine; cladibrine; cyclophosphamine; cytarabine; decarazine; docetaxel; idarubicin; ifosfamide; irinotecan; fludarabine; mitosmycin; mitoxane; mitoxantrone; topotecan; vinorelbine; adriamycin; mithram; imiquimod; alemtuzmab; exemestane; bevacizumab; cetuximab; azacitidine; clofarabine; decitabine; desatinib; dexrazoxane; docetaxel; epirubicin; oxaliplatin; erlotinib; raloxifene; fulvestrant; letrozole; gefitinib; gemtuzumab; trastuzumab; gefitinib; ixabepilone; lapatinib; lenalidomide; aminolevulinic acid; temozolomide; nelarabine; sorafenib; nilotinib; pegaspargase; pemetrexed; rituximab; dasatinib; thalidomide; bexarotene; temsirolimus; bortezomib; vorinostat; capecitabine; zoledronic acid; anastrozole; sunitinib; aprepitant and nelarabine, or a pharmaceutically acceptable salt thereof.

Additional pharmaceutically active agents that can be used in the treatment of cancers and that can be used in combination with one or more methods of the present invention include: epoetin alfa; darbepoetin alfa; panitumumab; pegfilgrastim; palifermin; filgrastim; denosumab; ancestim; AMG 102; AMG 386; AMG 479; AMG 655; AMG 745; AMG 951; and AMG 706, or a pharmaceutically acceptable salt thereof.

In addition, the substances of the present invention can be used in combination with other agents that can be used to treat cancer such as acemannan; aclarubicin; aldesleukin; alitretinoin; amifostine; amrubicin; amsacrine; anagrelide; arglabin; arsenic trioxide; BAM 002 (Novelos); bicalutamide; broxuridine; celmoleukin; cetrorelix; cladribine; clotrimazole; DA 3030 (Dong-A); daclizumab; denileukin diftitox; deslorelin; dilazep; docosanol; doxercalciferol; doxifluridine; bromocriptine; cytarabine; HIT diclofenac; interferon alfa; tretinoin; edelfosine; edrecolomab; eflornithine; emitefur; epirubicin; epoetin beta; etoposide phosphate; exisulind; fadrozole; finasteride; fludarabine phosphate; formestane; fotemustine; gallium nitrate; gemtuzumab zogamicin; gimeracil/oteracil/tegafur combination; glycopine; goserelin; heptaplatin; human chorionic gonadotropin; human fetal alpha fetoprotein; ibandronic acid; interferon alfa; interferon alfa natural; interferon alfa-2; interferon alfa-2a; interferon alfa-2b; interferon alfa-N1; interferon alfa-n3; interferon alfacon-1; interferon alpha natural; interferon beta; interferon beta-1a; interferon beta-1b; interferon gamma natural; interferon gamma-1a; interferon gamma-1b; interleukin-1 beta; iobenguane; irsogladine; lanreotide; LC 9018 (Yakult); leflunomide; lenograstim; lentinan sulfate; letrozole; leukocyte alpha interferon; leuprorelin; levamisole + fluorouracil; liarozole; lobaplatin; lonidamine; lovastatin; masoprocol; melarsoprol; metoclopramide; mifepristone; miltefosine; mirimostim; mismatched double stranded RNA; mitoguazone; mitolactol; mitoxantrone; molgramostim; nafarelin; naloxone + pentazocine; nartograstim; nedaplatin; nilutamide; noscapine; novel erythropoiesis stimulating protein; NSC 631570 octreotide; oprelvekin; osaterone; paclitaxel; pamidronic acid; peginterferon alfa-2b; pentosan polysulfate sodium; pentostatin; picibanil; pirarubicin; rabbit antithymocyte polyclonal antibody; polyethylene glycol interferon alfa-2a; porfimer sodium; raltitrexed; rasburicase; rhenium Re 186 etidronate; RII retinamide; romurtide; samarium (153 Sm) lexidronam; sargramostim; sizofiran; sobuzoxane; sonermin; strontium-89 chloride; suramin; tasonermin; tazarotene; tegafur; temoporfin; teniposide; tetrachlorodecaoxide; thymalfasin; thyrotropin alfa; toremifene; tositumomab-iodine 131; treosulfan; tretinoin; trilostane; trimetrexate; triptorelin; tumor necrosis factor alpha natural; ubenimex; bladder cancer vaccine; Maruyama vaccine; melanoma lysate vaccine; valrubicin; verteporfin; virulizin; zinostatin stimalamer; abarelix; AE 941 (Aeterna); ambamustine; antisense oligonucleotide; bcl-2 (Genta); APC 8015 (Dendreon); dexaminoglutethimide; diaziquone; EL 532 (Elan); EM 800 (Endorecherche); eniluracil; etanidazole; fenretinide; filgrastim SD01 (Amgen); galocitabine; gastrin 17 immunogen; HLA-B7 gene therapy (Vical); granulocyte macrophage colony stimulating factor; histamine dihydrochloride; ibritumomab tiuxetan; ilomastat; IM 862 (Cytran); interleukin-2; iproxifene; LDI 200 (Milkhaus); leridistim; lintuzumab; CA 125 monoclonal antibody(MAb) (Biomira); cancer MAb (Japan Pharmaceutical Development); HER-2 and Fc MAb (Medarex); idiotypic 105AD7 MAb (CRC Technology); idiotypic CEA MAb (Trilex); LYM-1-iodine 131 MAb (Techniclone); polymorphic epithelial mucin-yttrium 90 MAb (Antisoma); marimastat; menogaril; mitumomab; motexafin gadolinium; MX 6 (Galderma); nolatrexed; P 30 protein; pegvisomant; porfiromycin; prinomastat; RL 0903 (Shire); rubitecan; satraplatin; sodium phenylacetate; sparfosic acid; SRL 172 (SR Pharma); SU 5416 (SUGEN); TA 077 (Tanabe); tetrathiomolybdate; thaliblastine; thrombopoietin; tin ethyl etiopurpurin; tirapazamine; cancer vaccine (Biomira); melanoma vaccine (New York University); melanoma vaccine (Sloan Kettering Institute); melanoma oncolysate vaccine (New York Medical College); viral melanoma cell lysates vaccine (Royal Newcastle Hospital); or valspodar. The agents recited above may also be administered as pharmaceutically acceptable salts when appropriate.

### Pharmaceutical formulations and administration methods

Pharmaceutical formulations of the agents described above typically comprise a pharmaceutically acceptable carrier or diluent appropriate for a particular method of administration. The term "pharmaceutically acceptable" means that the referenced pharmaceutically active substance, or a particular excipent, are suitable for administration to a patient. Patients include animals, particularly mammals such as dogs, cats, cows, horses, sheep, and humans.

Compositions suitable for parenteral administration conveniently comprise a sterile aqueous preparation of a compound or composition of the invention, which is preferably isotonic with the blood of the recipient. This aqueous preparation may be formulated according to known methods using suitable dispersing or wetting agents, emulsifying and suspending agents. Various antibacterial and antifungal agents, for example, parabens, chlorobutanol, phenol, and sorbic acid also may be included. The sterile injectable preparation also may be a sterile injectable solution or suspension in a non-toxic parenterally-acceptable diluent or solvent, for example, as a solution in 1, 3-butane diol. Among the acceptable vehicles and solvents that may be employed are water, Ringer's solution, and isotonic sodium chloride solution. In addition, sterile, fixed oils are conventionally employed as a solvent or suspending medium. For this purpose any bland fixed oil may be employed including synthetic mono-or di-glycerides. In addition, fatty acids such as oleic acid may be used in the preparation of injectables. Prolonged absorption of the injectable pharmaceutical form can be brought about by the use of agents delaying absorption, for example, aluminum monostearate and gelatin. Carrier formulation suitable for subcutaneous, intravenous, intramuscular, etc. administrations can be found in Remington's Pharmaceutical Sciences, Mack Publishing Co., Easton, PA.

Solid dosage forms for oral administration include capsules, tablets, pills, powders, and granules. In such solid dosage forms, a compound or a mixture of compounds is admixed with at least one inert pharmaceutically acceptable excipient. The term "excipient" means any pharmaceutically acceptable additive, carrier, diluent, adjuvant, or other ingredient, other than the active pharmaceutical ingredient (API), which is typically included for formulation and/or administration to a patient. Suitable excipients include (a) fillers or extenders, as for example, starches, lactose, sucrose, glucose, mannitol, and silicic acid, (b) binders, as for example, carboxymethylcellulose, alignates, gelatin, polyvinylpyrrolidone, sucrose, and acacia, (c) humectants, as for example, glycerol, (d) disintegrating agents, as for example, agar-agar, calcium carbonate, potato or tapioca starch, alginic acid, certain complex silicates, and sodium carbonate, (e) solution retarders, as for example paraffin, (f) absorption accelerators, as for example, quaternary ammonium compounds, (g) wetting agents, as for example, cetyl alcohol, and glycerol monostearate, (h) adsorbents, as for example, kaolin and bentonite, and (i) lubricants, as for example, talc, calcium stearate, magnesium stearate, solid polyethylene glycols, sodium lauryl sulfate, or mixtures thereof. In the case of capsules, tablets, and pills, the dosage forms may also comprise buffering agents. Solid dosage forms such as tablets, dragees, capsules, pills, and granules also can be prepared with coatings and shells, such as enteric coatings and others well known in the art. The solid dosage form also may contain opacifying agents, and can also be of such composition that they release the active compound or compounds in a certain part of the intestinal tract in a delayed manner. Examples of embedding compositions which can be used are polymeric substances and waxes. The active compounds can also be in micro-encapsulated form, if appropriate, with one or more of the above-mentioned excipients. Such solid dosage forms may generally contain from 1% to 95% (w/w) of active compounds. In certain embodiments, active compounds range from 5% to 70% (w/w).

Liquid dosage forms for oral administration include pharmaceutically acceptable emulsions, solutions, suspensions, syrups, and elixirs. In addition to the compound or the mixture of compounds, the liquid dosage forms may contain inert diluents commonly used in the art, such as water or other solvents, solubilizing agents and emulsifiers, as for example, ethyl alcohol, isopropyl alcohol, ethyl carbonate, ethyl acetate, benzyl alcohol, benzyl benzoate, propyleneglycol, 1,3-butyleneglycol, dimethylformamide, oils, in particular, cottonseed oil, groundnut oil, corn germ oil, olive oil, castor oil and sesame oil, glycerol, tetrahydrofurfuryl alcohol, polyethylene glycols and fatty acid esters of sorbitan or mixtures of these substances. Besides such inert diluents, the composition can also include adjuvants, such as wetting agents, emulsifying and suspending agents, sweetening, flavoring, and perfuming agents.

Compositions for rectal administrations are preferably suppositories which can be prepared by mixing compounds used in the present invention with suitable non-irritating excipients or carriers such as cocoa butter, polyethylene glycol or a low-melting, suppository wax, which are solid at ordinary temperatures but liquid at body temperature and therefore, melt in the rectum or vaginal cavity and release the active compound.

Dosage forms for topical administration of a compound used in this invention include ointments, powders, sprays, and inhalants. An active compound is admixed under sterile conditions with a physiologically acceptable carrier and any preservatives, buffers, or propellants as may be required. Ophthalmic formulations, eye ointments, powders, and solutions are also contemplated as being within the scope of this invention.

Compounds and compositions used in the present invention also may benefit from a variety of delivery systems, including time-released, delayed release or sustained release delivery systems. Such option may be particularly beneficial when the compounds and composition are used in conjunction with other treatment methods as described in more detail below.

Many types of release delivery systems are available and known to those of ordinary skill in the art. They include polymer base systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Pat. No. 5,075,109. Delivery systems also include non-polymer systems that are: lipids including sterols such as cholesterol, cholesterol esters and fatty acids or neutral fats such as mono-di-and tri-glycerides; hydrogel release systems; sylastic systems; peptide based systems; wax coatings; compressed tablets using conventional binders and excipients; partially fused implants; and the like. Specific examples include, but are not limited to: (a) erosional systems in which the active compound is contained in a form within a matrix such as those described in U.S. Pat. Nos. 4,452,775, 4,667,014, 4,748,034 and 5,239,660 and (b) diffusional systems in which an active component permeates at a controlled rate from a polymer such as described in U.S. Pat. Nos. 3,832,253, and 3,854,480. In addition, pump-based hardware delivery systems can be used, some of which are adapted for implantation.

Use of a long-term sustained release implant may be desirable. Long-term release, as used herein, means that the implant is constructed and arranged to deliver therapeutic levels of the active compound for at least 30 days, and preferably 60 days. Long-term sustained release implants are well-known to those of ordinary skill in the art and include some of the release systems described above.

Pharmaceutical formulations can be administered in the conventional manner by routes including systemic, topical, and oral routes. For example, administration can be, but is not limited to, parenteral, subcutaneous, intravenous, intramuscular, intraperitoneal, transdermal, oral, buccal, intravaginal, or ocular routes, by inhalation, by depot injections, or by implants. Administration can be via bolus or by infusion (ranging from, for example, 15-30 minutes up to 7 days). Specific modes of administration will depend on the indication being treated and other factors including the particular compound being administered. The amount of compound to be administered is that amount which is therapeutically effective. The term "therapeutically effective amount" means an amount of a compound or biologic or combination thereof that ameliorates, attenuates or eliminates one or more symptom of a particular disease or condition, or prevents or delays the onset of one of more symptom of a particular disease or condition.

The dosage to be administered will depend on the characteristics of the subject being treated, *e.g*., the particular patient treated, age, weight, health, types of concurrent treatment, if any. Doses of IAP inhibitors range from 0.5 to 50 mg/kg. Doses of TNFα inhibitors range from 0.1 to 50 mg/kg. Doses of TRAIL receptor agonists range from 0.1 to 75 mg/kg. Frequency of treatments (*e.g*., daily, weekly, etc.) can be determined by one of skill in the art (*e.g*., by the clinician) by routine experimentation.

Methods of the invention can be used to induce apoptosis to treat cancer, including both solid and non-solid tumors, as well as various tumors. "Treat," as used herein, means to reduce, stabilize, or inhibit progression of a symptom, such as tumor size. Cancers which can be treated with IAP inhibitors include, but are not limited to, one or more of the following: lung adenocarcinoma, pancreatic cancer, colon cancer, ovarian cancer, breast cancer, mesothelioma, peripheral neuroma, bladder cancer, glioblastoma, melanoma, adrenocortical carcinoma, AIDS-related lymphoma, anal cancer, bladder cancer, meningioma, glioma, astrocytoma, breast cancer, cervical cancer, chronic myeloproliferative disorders (e.g., chronic lymphocytic leukemia, chronic myelogenous leukemia), colon cancer, endocrine cancers, endometrial cancer, ependymoma, esophageal cancer, Ewing's sarcoma, extracranial germ cell tumors, extragonadal germ cell tumors, extrahepatic bile duct cancer, gallbladder cancer, gastric cancer, gastrointestinal carcinoid tumors, gestational trophoblastic tumors, hairy cell leukemia, Hodgkin lymphoma, non-Hodgkin lymphoma, hypopharyngeal cancer, intraocular melanoma, islet cell carcinoma, Kaposi sarcoma, laryngeal cancer, leukemia, acute lymphoblastic leukemia, acute myeloid leukemia, lip cancer, oral cavity cancer, liver cancer, male breast cancer, malignant mesothelioma, medulloblastoma, melanoma, Merkel cell carcinoma, metastatic squamous neck cancer, multiple myeloma and other plasma cell neoplasms, mycosis fungoides and the Sézary syndrome, myelodysplastic syndromes, nasopharyngeal cancer, neuroblastoma, non-small cell lung cancer, small cell lung cancer, oropharyngeal cancer, bone cancers, including osteosarcoma and malignant fibrous histiocytoma of bone, ovarian epithelial cancer, ovarian germ cell tumors, ovarian low malignant potential tumors, pancreatic cancer, paranasal sinus cancer, parathyroid cancer, penile cancer, pheochromocytoma, pituitary tumors, prostate cancer, rectal cancer, renal cell cancer, retinoblastoma, rhabdomyosarcoma, salivary gland cancer, skin cancer, small intestine cancer, soft tissue sarcoma, supratentorial primitive neuroectodermal tumors, pineoblastoma, testicular cancer, thymoma, thymic carcinoma, thyroid cancer, transitional cell cancer of the renal pelvis and ureter, urethral cancer, uterine sarcoma, vaginal cancer, vulvar cancer, and Wilm's tumor and other childhood kidney tumors.

The TNFα inhibitor typically is administered as a pretreatment so that, preferably, maximal TNF inhibition is achieved before the IAP inhibitor and TRAIL receptor agonist are administered. A variety of assays for measuring TNF inhibition are known in the art and can be used to monitor TNF inhibition. These assays include, but are not limited to, immunoassays and competitive binding assays. See US 2008/0167223.

Pretreatment with the TNFα inhibitor typically occurs between about 1 to about 24 hours before administration of the IAP inhibitor and TRAIL receptor agonist, however very fast-acting TNF inhibitors could be administered essentially simultaneously with the IAP inhibitor and TRAIL receptor agonist (coadministered). In some embodiments, the TNFα inhibitor can be administered after administration of the IAP inhibitor and TRAIL receptor agonist if undesired effects associated with elevated TNFα are observed which could be ameliorated by administration of a TNFα inhibitor. *See* Blick et al., Cancer Res. 47, 2986-89, 1987; Kurzrock et al., J. Clin. Oncol. 6, 1328-34, 1988 ; Spriggs et al., J. Nat'l. Cancer Inst. 80, 1039-44, 1988; and Widenmann et al., J. Cancer Res. Clin. Oncol. 115, 189-92, 1989.

The IAP inhibitor and TRAIL receptor agonist can be administered separately or can be present in the same pharmaceutical composition. In some embodiments the two agents are present in different pharmaceutical compositions and can be administered essentially simultaneously or within several minutes of each other.

### EXAMPLE 1

### Inhibition of TNFα reduces IAP inhibitor-induced elevation of caspase 3/7 activity in serum of naïve mice

The IAP inhibitor, "compound X," has the following structural formula: 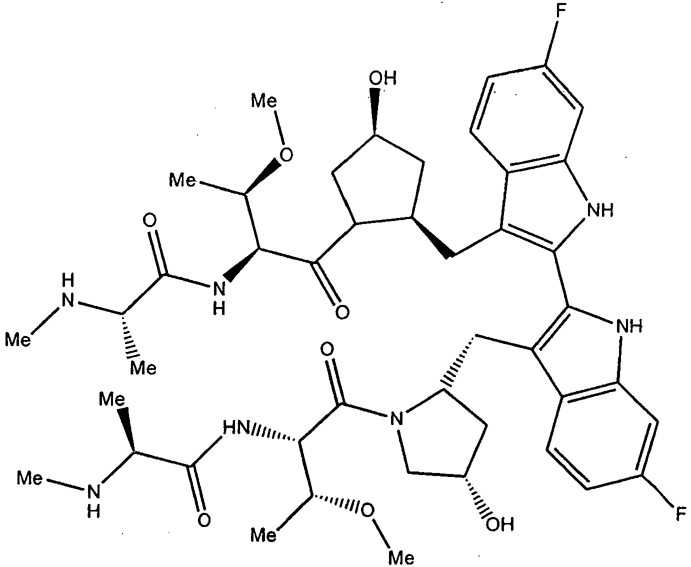 in which "Me" is methyl. Compound X and its synthesis are described in US 2006/0194741 (in which compound X is compound no. 116). Compound X results in elevated caspase 3/7 in the serum of naïve (non-tumor bearing) mice. This experiment demonstrates that induction of caspase 3/7 activity by the IAP inhibitor can be blocked by inhibition of TNFα.

Non-tumor bearing NCr nude mice (Taconic Farms, Inc., Germantown, NY) were injected intraperitoneally according to the following scheme:

| **Mouse Group** | **pre-treatment** | **Treatment** |
|---|---|---|
| 1 | con-FC (500 µg) | vehicle |
| 2 | con-FC (500 µg) | compound X (15 mg/kg) |
| 3 | PEG-huTNFR1-FC (100 µg) | compound X (15 mg/kg) |
| 4 | PEG-huTNFR1-FC (200 µg) | compound X (15 mg/kg) |
| 5 | PEG-huTNFR1-FC (300 µg) | compound X (15 mg/kg) |
| 6 | PEG-huTNFRI-FC (400 µg) | compound X (15 mg/kg) |
| 7 | PEG-huTNFRI-FC (500 µg) | compound X (15 mg/kg) |
| 8 | PEG-huTNFR1-FC (500 µg) | vehicle |
| 9 | anti-muTNFα (500 µg) | vehicle |
| 10 | anti-muTNFα (500 µg) | compound X (15 mg/kg) |
| 11 | hamster IgG (500 µg) | vehicle |
| 12 | hamster IgG (500 µg) | compound X (15 mg/kg) |

In the table above, "anti-muTNFα" is a monoclonal antibody generated against murine TNFα, and "PEG-huTNFR1-FC" is a PEGylated human fusion of TNFR1 with an immunoglobulin FC portion; "con-FC" is the FC portion alone (control). Fusions of TNFR1 and an immunoglobulin FC portion can be prepared using methods described in U.S. Patents 5,808,029; 5,605,690; or 6,143,866. Methods of PEGylation are well known in the art. All injection volumes were 100 µl, and each group consisted of 3 mice.

The pretreatment injections were administered two hours before the treatment injections. Six hours after the treatment injections, mice were sacrificed, and serum was collected and stored at -80 °C. Caspase 3/7 activity in the serum samples was assessed using the CASPASE-GLO® 3/7 Assay System (Promega, Madison, WI) according to the manufacturer's recommendations. This assay provides a proluminescent caspase-3/7 aminoluciferin substrate (Asp-Glu-Val-Asp) and a thermostable luciferase in a reagent optimized for caspase-3/7 activity, luciferase activity, and cell lysis. Adding the reagent causes cell lysis, followed by caspase cleavage of the substrate, which liberates free aminoluciferin; the free amino luciferin is consumed by the luciferase, generating a luminescent signal which is proportional to caspase-3/7 activity. Relative light units (RLU) were determined using a Wallac Envision platereader (Perkin Elmer).

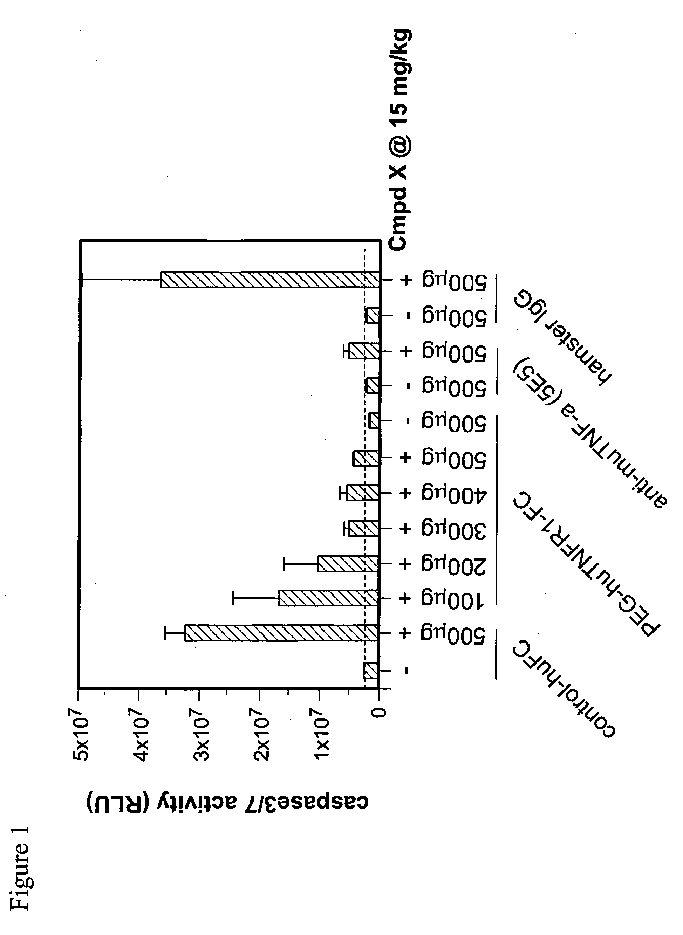

The results are shown in **FIG. 1****.** Intraperitoneal injection of IAP inhibitor compound X resulted in elevated caspase 3/7 activity in the serum of naïve mice (groups 2 and 12). However, caspase 3/7 activity was drastically reduced if mice were injected first with a neutralizing antibody to murine TNFα (group 10). In addition, administration of an increasing dose of the recombinant protein PEG-huTNFR1-FC also inhibited the compound X-induced caspase 3/7 activity in the serum of naïve mice (groups 3-7).

These experiments demonstrate that IAP inhibitor-induced elevation of caspase 3/7 activity in the serum of naïve mice is reduced by inhibition of TNFα.

### EXAMPLE 2

### Effects of a lower dose of an IAP inhibitor on various measures of apoptosis in wild-type vs TNFR-deficient mice

Inhibition of a cIAP causes cell death by up-regulation of TNFα production. Thus, TNFR (p55/p75) double knock-out (DKO) mice should be less prone to apoptosis in response to cIAP inhibition compared to wild-type (WT) mice. To test this hypothesis, the IAP inhibitor compound X was administered to WT and to p55/p75TNFR double knock-out (TNFR DKO) mice. Apoptotic activity was assessed by measuring serum caspase 3 levels, monitoring blood cell counts, and by histological analyses of apoptosis in specific tissues by immunohistochemistry.

C57B1/6 WT or TNFR DKO mice approximately six weeks old were obtained from Taconic Farms, Inc.. Compound X was formulated in 12.5% CAPTISOL^{®}, an anionically charged sulfobutyl ether ß-cyclodextrin, and was administered to the WT or TNFR DKO mice at 30 mg/kg, intraperitoneally, daily for five consecutive days. Groups included WT or TNFR DKO mice treated with vehicle (n=5) or compound X (n=10). Body weights were monitored throughout the study from days 0 to 8. Five mice were necropsied from the compound X-treated groups on day 5 approximately 5 hours after the 5^{th} daily dose to assess serum caspase 3 levels, complete blood counts (CBC), and tissue-specific apoptosis. Whole blood was collected via cardiac puncture, and serum was separated according to standard procedures. Serum caspase 3 levels were quantitated using the Promega CASPASE-GLO® 3/7 Assay System described above.

The remaining mice were allowed to recover and were necropsied on day 9 (5 mice each from the vehicle- and compound X-treated groups). Tissues collected included skin, heart, lung, thymus, stomach, small intestine, and colon. Tissues were fixed in 10% neutral buffered formalin, embedded in paraffin, sectioned at 5 µm, stained with hematoxylin and eosin, and examined with transmitted light microscopy. Histopathological lesions were identified and subjectively graded on the following scale: 0 (no change), 1 (negligible change), 2 (mild change), 3 (moderate change), 4 (marked change), and 5 (severe change). The severity grade for each lesion category was averaged for 3 animals per group.

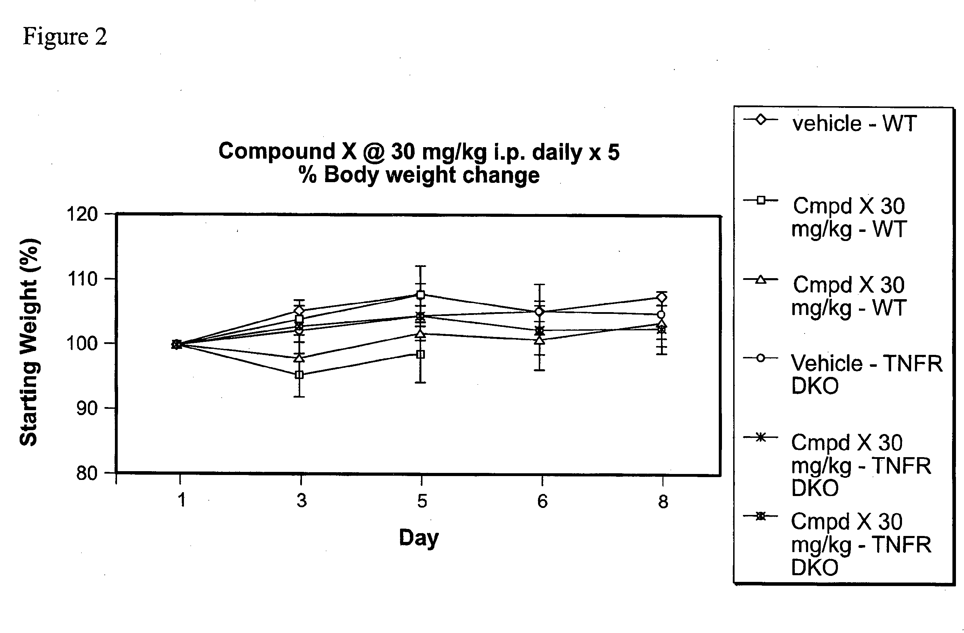

Treatment with compound X at this dose resulted in minimal body weight loss in both WT and TNFR DKO mice **(****FIG. 2****).** C57B1/6 mice are known to be less sensitive to a variety of agents than some other mouse strains. Their tolerance for compound X, measured by body weight loss, appears to be higher than other strains (>10% body weight loss was observed in Balb/c and Ncr nude mice treated as described above with compound X).

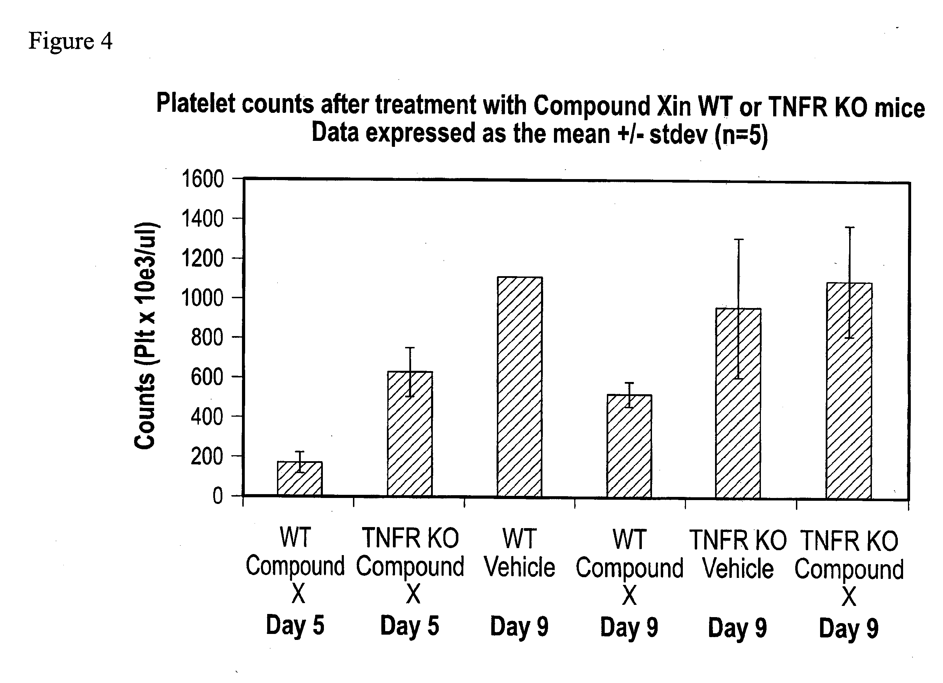

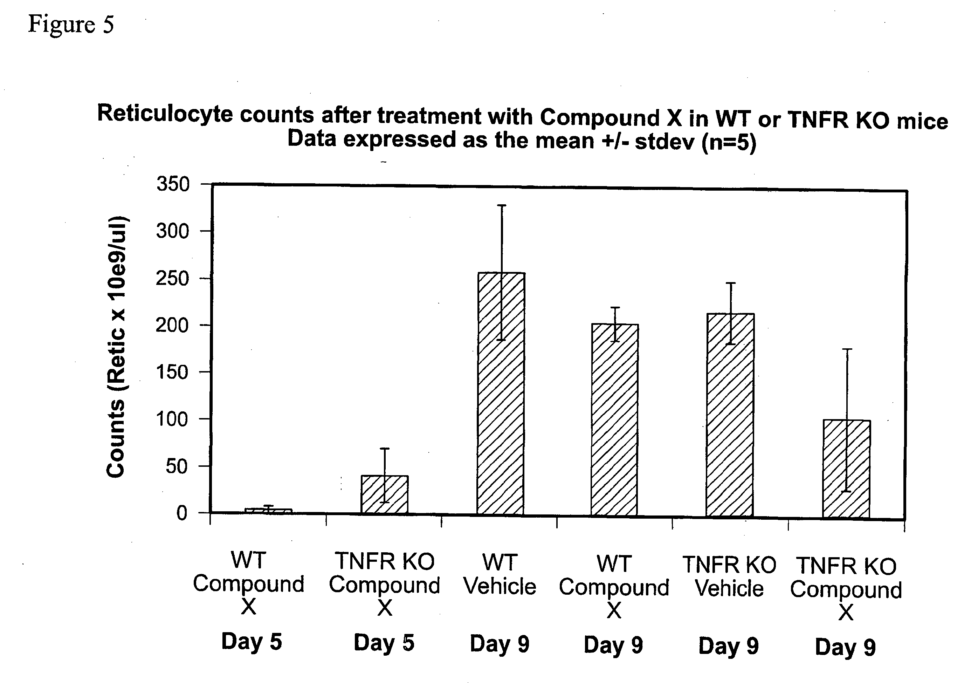

CBC data suggest a reduction in platelets and reticulocytes at day 5, recovering by day 9 in both WT and TNFR DKO mice, with the WT mice being more affected than the TNFR DKO mice at day 5 **(****FIGS. 4**,**5**).

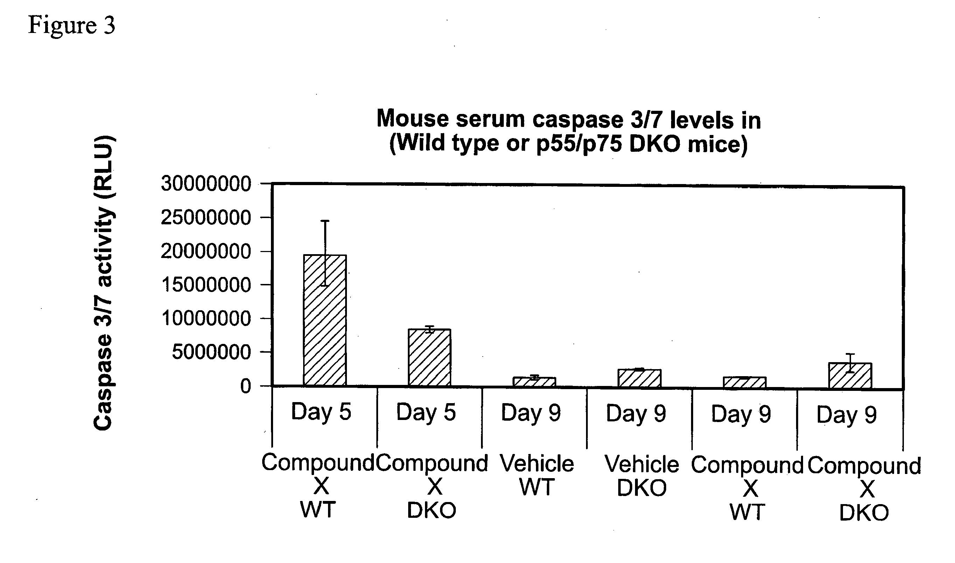

Serum caspase 3 levels are higher in WT mice at day 5 compared to the TNFR DKO mice, indicating that the TNF signaling pathway is important in inducing apoptosis after cIAP inhibition. Serum caspase 3 levels returned to baseline in both the WT and TNFR DKO compound X-treated mice by day 9. See **FIG. 3****.**

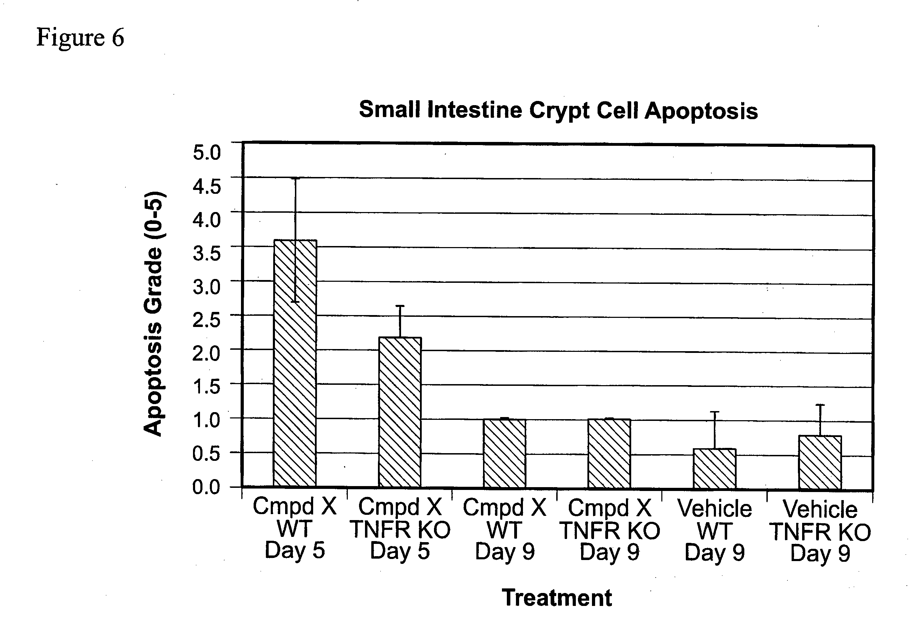

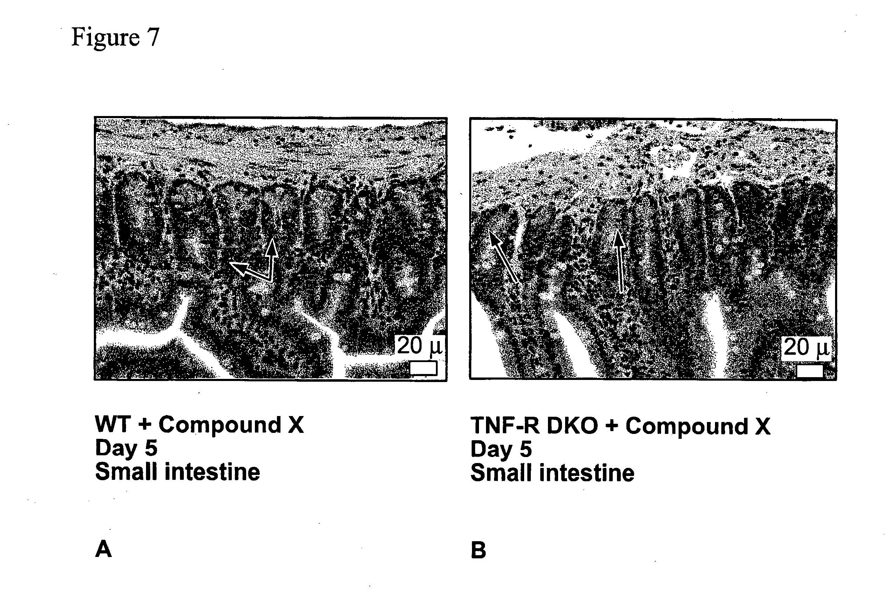

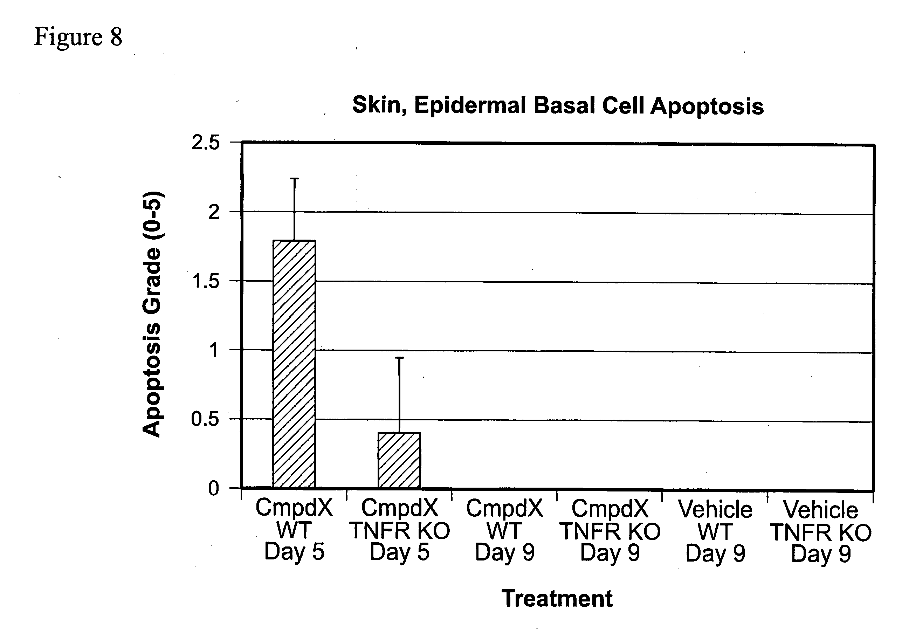

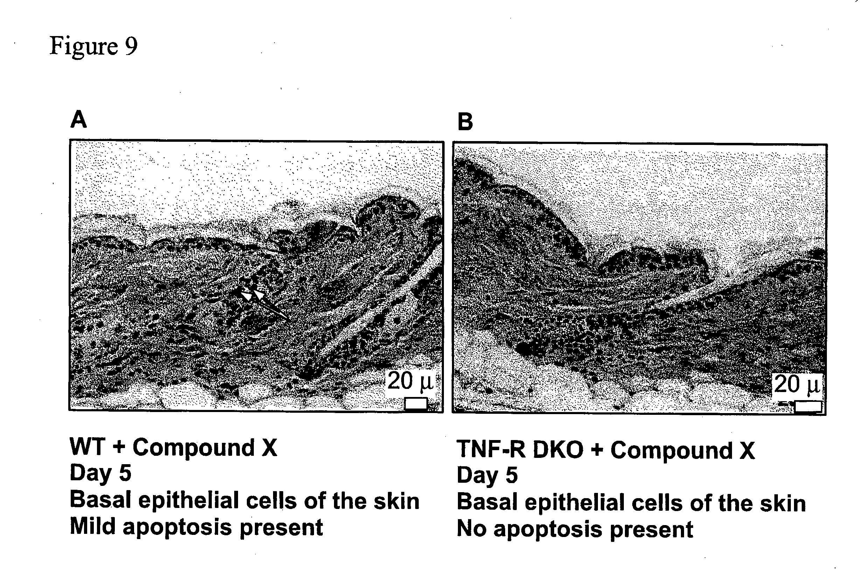

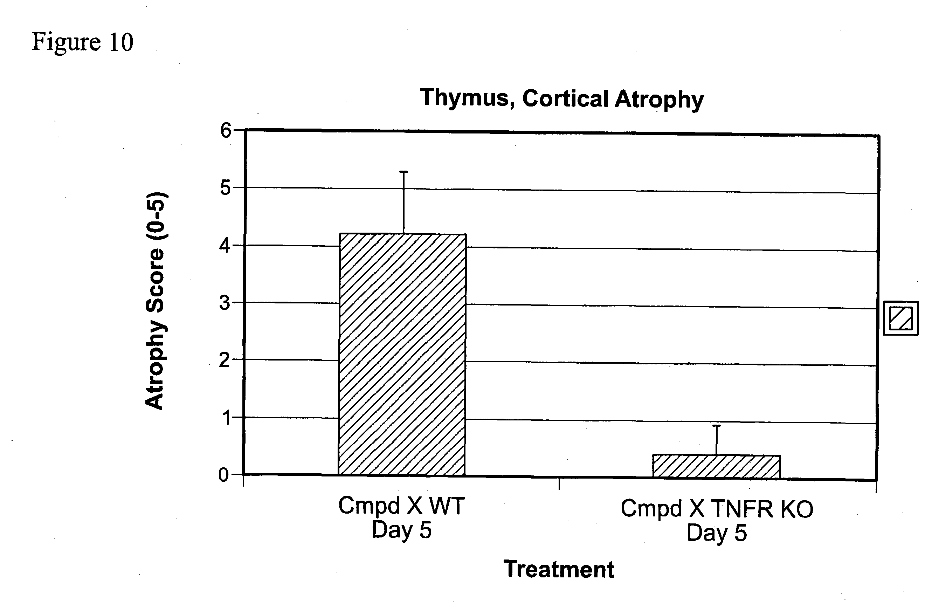

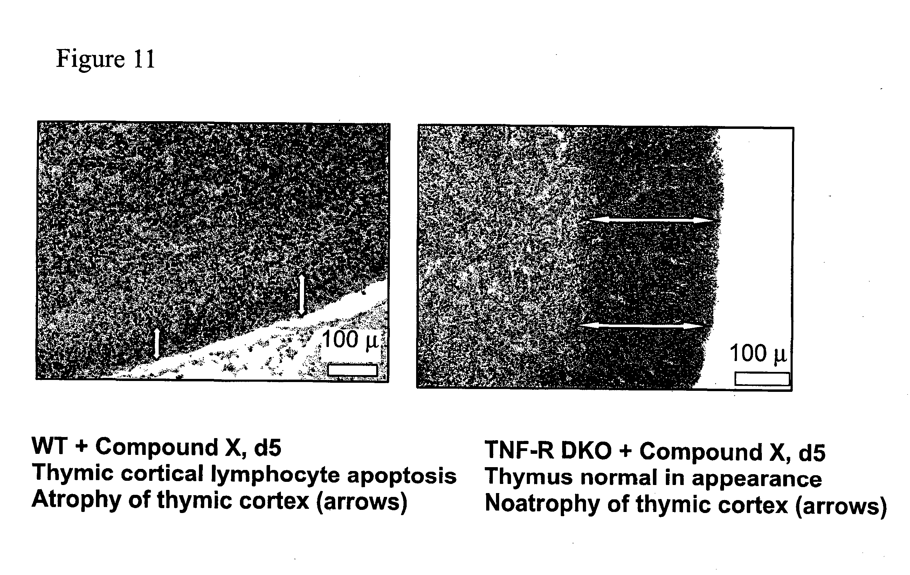

Treatment with compound X induced apoptosis of intestinal tract epithelium **(****FIGS. 6**, **7**) and epidermal basal cells **(****FIGS. 8**, **9**) and caused degeneration and apoptosis of lymphocytes of the thymus **(****FIGS. 10**, **11**). Indirect evidence of the degree of extramedullary hematopoiesis in the spleen of recovery animals indicates that hematopoietic cells of the bone marrow were also degenerated following treatment. The effects caused by compound X treatment were significantly less severe or were effectively prevented (thymic cortical atrophy and hematopoietic cell degeneration) in TNFR DKO mice than in WT mice.

### EXAMPLE 3

### Effects of a higher dose of an IAP inhibitor on various measures of apoptosis in wild-type vs TNFR-deficient mice

C57B1/6 mice are known to be less sensitive to a variety of agents than some other mouse strains. Their tolerance for compound X, measured by body weight loss, appears to be higher than other strains as discussed in Example 2. As a result, the study was repeated with a higher dose of compound X in order to elicit a more robust response in the C57Bl/6 mice similar to what has been previously observed in other mouse strains.

In this example, the IAP inhibitor compound X was administered to wild-type (WT) and to p55/p75TNFR double knock-out (TNFR DKO) mice at 40 mg/kg qdx5 i.p. compared to 30 mg/kg in Example 2, above. TNFα-mediated toxicity was assessed by measuring serum caspase 3 levels and by monitoring blood cell counts.

C57B1/6 WT or TNFR DKO mice approximately sixteen weeks old were obtained from Taconic Farms, Inc.. Compound X was formulated in 12.5% CAPTISOL®, an anionically charged sulfobutyl ether ß-cyclodextrin, and was administered to the WT or TNFR DKO mice at 40 mg/kg, intraperitoneally, daily for five consecutive days. Groups included WT or TNFR DKO mice treated with vehicle (n=13) or compound X (n=13 or 14). Body weights were monitored throughout the study from days 1 to 5. All mice were necropsied on day 5 approximately 4 to 6 hours after the 5th daily dose to assess serum caspase 3 levels, complete blood counts (CBC), and tissue-specific apoptosis. Whole blood was collected via cardiac puncture, and serum was separated according to standard procedures. Serum caspase 3 levels were quantitated using the Promega CASPASE-GLO® 3/7 Assay System as previously described.

Treatment with compound X at 40 mg/kg resulted in a 10% body weight loss in WT mice compared to 0% in TNFR DKO mice **(****FIG. 12A****).** The data suggest that signaling through TNF receptors contributes to compound X-induced body weight loss.

Serum caspase 3 levels are elevated in WT mice compared to the TNFR DKO mice, indicating that the TNF signaling pathway is important in inducing apoptosis after cIAP inhibition **(****FIG. 12B****).**

CBC data indicate a marked reduction in reticulocytes, platelets and white blood cells at day 5 in the WT mice compared to the TNFR DKO mice. Red blood cell and hemogoblin counts are relatively unchanged after treatment with compound X in both WT and TNFR DKO mice **(****FIG. 13A-C**). Neutrophils appear to be increased and lymphocytes are decreased in both the WT and TNFR DKO mice after compound X treatment relative to the vehicle controls **(****FIG. 13D****).**

### EXAMPLE 4

### Demonstration that inhibition of TNFα does not block TRAIL receptor agonist synergy with IAP inhibitors in vitro

Cells were seeded in flat-bottom 96-well plates (clear bottom, white walls) at a concentration of 1 x 10⁴ cells/well and incubated overnight at 37 °C, 5% CO₂. The following day, cells were stimulated with TRAIL at a concentration ranging from 0.24 ng/ml to 1 µg/ml (4-fold dilutions) in combination with IAP inhibitor compound X at a concentration ranging from 0.01 nM to 10 mM (10-fold dilutions).

A blocking antibody to human TNFα (R&D Systems, Minneapolis, MN) was added to some cultures at a final concentration of 5 µg/ml two hours before the addition of the combination of TRAIL and compound X. Final culture volumes were 100 µl.

After 48 hours, cell viability was determined using the ATPLITE^{™} assay, an adenosine triphosphate (ATP) monitoring system based on firefly *(Photinus pyralis)* luciferase (Perkin Elmer, Waltham, MA) according to the manufacturer's recommendations. Relative light units (RLU) were measured using an LJL Biosystems Analyst HT plate reader (Molecular Devices, Sunnyvale, CA). The percent of surviving cells was calculated as follows: (experimental RLU / control RLU) x 100. The control RLU was determined by testing untreated cells. The results are shown in **FIG. 14****.**

As shown in **FIG. 14A****,** the colon carcinoma cell line HCT116 is resistant to compound X-induced cell death but shows some sensitivity to TRAIL, because only 40% of the cells survive at high concentrations (1 µg/ml) of TRAIL. However, when TRAIL and compound X are administered essentially simultaneously there appears to be an enhanced effect on cell killing. This synergistic effect on cell killing is not blocked in the presence of a neutralizing antibody to human TNFα **(****FIG. 14B****).**

**FIG. 14C** demonstrates that the melanoma cell line A375 is partialy resistant to single agent killing induced by either compound X or TRAIL; however, the combination of TRAIL and compound X induces very efficient cell killing. Similar to the HCT116 cell line, this synergistic effect is not altered in the presence of a neutralizing antibody to human TNFα **(****FIG. 14D****).**

The ovarian carcinoma cell line SKOV-3 is resistant to TRAIL-induced cell death but appears to have some sensitivity to compound X-induced cell death **(****FIG. 14E****).** This single agent efficacy is mediated via TNFα, because the presence of a neutralizing antibody to human TNFα blocks compound X-induced cell killing **(****FIG. 14F****).** Interestingly, when TRAIL and compound X are used together in the presence of a neutralizing antibody to human TNFα, the inhibition of compound X-induced cell killing due to the antibody is removed, and the combination exhibits potent cell killing. Similar results are obtained with the following cancer cell lines: Colo205 (human colon adenocarcinoma), MDA-MB-468 (human breast adenocarcinoma), HCT-15 (human colon adenocarcinoma), MDA-MB-231 (human breast adenocarcinoma; obtained from the American Type Culture Collection, ATCC), and MDA-MB-231 (substrain BB), which is a different substrain.

These results demonstrate that inhibition of TNFα does not block the enhanced anti-tumor activity of the combination of TRAIL and IAP inhibitors *in vitro.*

### EXAMPLE 5

### Treatment of mice with a TNFα inhibitor, an IAP inhibitor, and a TRAIL receptor agonist

This example reports the results of an experiment in which mice were treated with a TNFα inhibitor, an IAP inhibitor, and a TRAIL receptor agonist. The TNFα inhibitor was "TNFR1-FC" (see Example 1) The IAP inhibitor was compound X (see Example 1). The TRAIL receptor agonist was an agonistic antibody (antibody "M413," which is a mouse monoclonal antibody generated against human TRAIL receptor 2, or DR5; see Griffith et al., J. Immunol. 162, 2597-605, 1999) to assess the effects on tumor caspase activation, which can predict anti-tumor activity.

SKOV3 tumor bearing mice were treated as described on the graph in **FIG. 15****.** Mice receiving 500 µg total TNFR1-FC were pre-treated 2 hours in advance. Six hours after treatment with compound X (15 mg/kg), M413 (50 µg total), or both, mice were euthanized by CO₂ asphyxiation, and tumors were excised and snap frozen in liquid nitrogen. Tumors were homogenized with the Tissuelyser machine (Qiagen, Valencia, CA). Briefly, tumors were homogenized using 5 mm stainless steel beads (Qiagen) in RIPA lysis buffer (Thermo Scientific, Rockford, IL) containing protease inhibitors (Roche, Indianapolis, IN) in a ratio of approximately 1 µl of buffer/1 mg tissue. The lysates were run in the Tissuelyser for 90 seconds at 30 Hz per cycle for a total of 3-4 cycles until tumor tissue was completely dissociated. Lysates were then filtered through Qiashredder tubes (Qiagen) by centrifugation at 14,000 rpm for 10 min at 4° C to pellet debris. Tumor lysate supernatants were transferred to new tubes and protein concentrations were determined using the BCA assay according to the manufacturer's recommendations (Thermo).

Tumor lysates were analyzed for the presence of cleaved caspase-3 by the MSD cleaved caspase-3 assay according to the manufacturer's specifications (Meso Scale Discovery, Gaithersburg, MD). Relative light units (RLU) were measured using the Sector plate reader (Meso Scale Discovery). The results are shown in **FIG. 15****.** Vehicle treated mice are shown in Lane 1, which indicates the basal background level of activated caspase 3 in SKOV3 tumors.

As expected, treatment of SKOV3 tumors with compound X alone resulted in an increase in activated caspase 3 over the vehicle control (Lane 2). Treatment with the DR5 antibody M413 also increased caspase 3 activity over vehicle control, but not to the degree that compound X did (Lane 3). Combination of compound X with M413 was similar to the activity seen with compound X alone, suggesting that tumor caspase induction with compound X was already at a maximal level. Treatment of tumors with TNFR1-Fc alone (Lane 5) had no effect on tumor caspase activity. However, when TNFR1-Fc was added 2 hr before compound X, it partially blocked compound X-induced tumor caspase activity (compare Lane 6 to Lane 2). This suggests that TNF is required for compound X single agent induced tumor caspase activity in SKOV3 tumors and blocking TNF using TNFR1-Fc can diminish this effect.

It is possible that the partial block on compound X induced caspase activation with TNFR1-Fc observed was due to either a limiting amount of TNFR1-Fc or requirement for a longer pre-treatment time. TNFR1-Fc pre-treatment would not be expected to impact the effects of M413. Consistent with this, there was minimal reduction of tumor caspase activation in the presence of TNFR1-Fc and M413 (Lane 7). Pre-treatment of mice with TNFR1-Fc can block compound X single agent mediated SKOV3 tumor caspase activation, as shown in Lane 6. However, when the DR5 agonistic antibody M413 was added in combination with compound X in the presence of TNFR1-Fc, a maximal induction of tumor caspase activity was observed (Lane 8). This suggests that the block on compound X single agent activity (by blocking TNF) had no effect on the ability of compound X to cooperate with M413.

These data support the fact that compound X single agent activity and compound X cooperativity with a TRAIL receptor agonist such as M413 function via distinct pathways. These data also support the idea that a TNF blocker can function to allow for compound X-TRAIL receptor agonist anti-tumor activity *in vivo,* while reducing the possibility of unwanted effects.

The invention described herein lends itself to a method of marketing or selling an IAP inhibitor, a TNFα inhibitor, or a TRAIL receptor agonist that comprises disseminating information to prospective or actual patients or prescribers concerning the combination of therapies described herein. Such information may include, *e.g*., that undesired effects that may be associate with IAP inhibition therapy may be mitigated by administration of a lower dose of the IAP inhibitor while administering it in conjunction with a TNFα inhibitor and a TRAIL receptor agonist, as described above.

The foregoing description of various aspects of the invention has been presented for purposes of illustration and description. It is not intended to be exhaustive or to limit the invention to the precise form disclosed, and obviously, many modifications and variations are possible. Such modifications and variations that may be apparent to a person skilled in the art are intended to be included within the scope of the invention as defined by the accompanying claims.

## Claims

1. A combination of a TNFα inhibitor, an IAP inhibitor and a TRAIL receptor agonist for use in treating cancer, wherein the TNFα inhibitor is administered for a sufficient time to achieve inhibition of TNFα prior to administration of the IAP inhibitor and the TRAIL receptor agonist.

2. The combination for use according to claim 1 wherein the IAP inhibitor is a small molecule.

3. The combination for use according to claim 1 wherein the TNFα inhibitor is a small molecule.

4. The combination for use according to claim 1 wherein the TRAIL receptor agonist is a small molecule.

5. The combination for use according to claim 1 wherein the IAP inhibitor and the TRAIL receptor agonist are administered simultaneously.

6. The combination for use according to claim 1 wherein the IAP inhibitor and the TRAIL receptor agonist are administered sequentially.

7. The combination for use according to claim 1 wherein the cancer is selected from the group consisting of ovarian cancer, breast cancer, and colon cancer.

8. The combination for use according to claim 1 wherein the IAP inhibitor is a Smac mimetic.

9. The combination for use according to claim 1 wherein the TNFα inhibitor is an antibody.

10. The combination for use according to claim 9 wherein the antibody is selected from the group consisting of Infliximab and Adalimumab.

11. The combination for use according to claim 1 wherein the TNFα inhibitor is Etanercept.

12. The combination for use according to claim 1 wherein the TRAIL receptor agonist is an antibody.

13. The combination for use according to claim 12 wherein:
1) the antibody is a DR4 antibody;
2) the antibody is a DR5 antibody; or
3) the antibody is a DR4 antibody and a DR-5 antibody.

14. The combination for use according to claim 1 wherein the TRAIL receptor agonist is a soluble TRAIL polypeptide.

## Patentansprüche

1. Kombination aus einem TNFα-Hemmer, einem IAP-Blocker und einem Agonisten des TRAIL-Rezeptors zur Verwendung bei der Behandlung von Krebs, wobei der TNFα-Hemmer hinreichend lange verabreicht wird, um eine Hemmung von TNFα zu erreichen, bevor der IAP-Blocker und der Agonist des TRAIL-Rezeptors verabreicht werden.

2. Kombination zur Verwendung nach Anspruch 1, wobei es sich bei dem IAP-Blocker um ein kleines Molekül handelt.

3. Kombination zur Verwendung nach Anspruch 1, wobei es sich bei dem TNFα-Hemmer um ein kleines Molekül handelt.

4. Kombination zur Verwendung nach Anspruch 1, wobei es sich bei dem Agonisten des TRAIL-Rezeptors um ein kleines Molekül handelt.

5. Kombination zur Verwendung nach Anspruch 1, wobei der IAP-Blocker und der Agonist des TRAIL-Rezeptors gleichzeitig verabreicht werden.

6. Kombination zur Verwendung nach Anspruch 1, wobei der IAP-Blocker und der Agonist des TRAIL-Rezeptors nacheinander verabreicht werden.

7. Kombination zur Verwendung nach Anspruch 1, wobei die Krebserkrankung aus der aus Ovarialkarzinom, Brustkrebs und Kolonkarzinom bestehenden Gruppe ausgewählt ist.

8. Kombination zur Verwendung nach Anspruch 1, wobei es sich bei dem IAP-Blocker um ein Smac-Mimetikum handelt.

9. Kombination zur Verwendung nach Anspruch 1, wobei es sich bei dem TNFα-Hemmer um einen Antikörper handelt.

10. Kombination zur Verwendung nach Anspruch 9, wobei der Antikörper aus der aus Infliximab und Adalimumab bestehenden Gruppe ausgewählt ist.

11. Kombination zur Verwendung nach Anspruch 1, wobei es sich bei dem TNFα-Hemmer um Etanercept handelt.

12. Kombination zur Verwendung nach Anspruch 1, wobei es sich bei dem Agonisten des TRAIL-Rezeptors um einen Antikörper handelt.

13. Kombination zur Verwendung nach Anspruch 12, wobei:
1) es sich bei dem Antikörper um einen DR4-Antikörper handelt;
2) es sich bei dem Antikörper um einen DR5-Antikörper handelt; oder
3) es sich bei dem Antikörper um einen DR4-Antikörper und einen DR5-Antikörper handelt.

14. Kombination zur Verwendung nach Anspruch 1, wobei es sich bei dem Agonisten des TRAIL-Rezeptors um ein lösliches TRAIL-Polypeptid handelt.

## Revendications

1. Combinaison d'un inhibiteur du TNFα, d'un inhibiteur IAP et d'un agoniste du récepteur de TRAIL pour son utilisation dans le traitement du cancer, dans laquelle l'inhibiteur du TNFα est administré pendant une durée suffisante pour atteindre l'inhibition du TNFα avant l'administration de l'inhibiteur IAP et de l'agoniste du récepteur de TRAIL.

2. Combinaison pour son utilisation selon la revendication 1, dans laquelle l'inhibiteur IAP est une petite molécule.

3. Combinaison pour son utilisation selon la revendication 1, dans laquelle l'inhibiteur du TNFα est une petite molécule.

4. Combinaison pour son utilisation selon la revendication 1, dans laquelle l'agoniste du récepteur de TRAIL est une petite molécule.

5. Combinaison pour son utilisation selon la revendication 1, dans laquelle l'inhibiteur IAP et l'agoniste du récepteur de TRAIL sont administrés simultanément.

6. Combinaison pour son utilisation selon la revendication 1, dans laquelle l'inhibiteur IAP et l'agoniste du récepteur de TRAIL sont administrés séquentiellement.

7. Combinaison pour son utilisation selon la revendication 1, dans laquelle le cancer est sélectionné dans le groupe constitué du cancer des ovaires, du cancer du sein, et du cancer du côlon.

8. Combinaison pour son utilisation selon la revendication 1, dans laquelle l'inhibiteur IAP est un mimétique de Smac.

9. Combinaison pour son utilisation selon la revendication 1, dans laquelle l'inhibiteur du TNFα est un anticorps.

10. Combinaison pour son utilisation selon la revendication 9, dans laquelle l'anticorps est sélectionné dans le groupe constitué de l'infliximab et de l'adalimumab.

11. Combinaison pour son utilisation selon la revendication 1, dans laquelle l'inhibiteur du TNFα est l'étanercept.

12. Combinaison pour son utilisation selon la revendication 1, dans laquelle l'agoniste du récepteur de TRAIL est un anticorps.

13. Combinaison pour son utilisation selon la revendication 12, dans laquelle :
1) l'anticorps est un anticorps anti-DR4 ;
2) l'anticorps est un anticorps anti-DR5 ; ou
3) l'anticorps est un anticorps anti-DR4 et un anticorps anti-DR5.

14. Combinaison pour son utilisation selon la revendication 1, dans laquelle l'agoniste du récepteur de TRAIL est un polypeptide TRAIL soluble.
